(19) Europäisches Patentamt
European Patent Office
Office européen des brevets

(11) Veröffentlichungsnummer: **0 057 349**
**B1**

# (12) EUROPÄISCHE PATENTSCHRIFT

(45) Veröffentlichungstag der Patentschrift: **27.06.84**

(51) Int. Cl.³: **C 07 C 103/52**, C 07 H 19/08, C 12 P 21/02, A 61 K 37/02 // (C12P21/02, C12R1/465)

(21) Anmeldenummer: **82100121.1**

(22) Anmeldetag: **11.01.82**

**(54) Neues Peptid-Antibiotikum Komponente B, Verfahren zu seiner Herstellung sowie seine Verwendung in Arzneimitteln.**

(30) Priorität: **23.01.81 DE 3102137**

(43) Veröffentlichungstag der Anmeldung: **11.08.82 Patentblatt 82/32**

(45) Bekanntmachung des Hinweises auf die Patenterteilung: **27.06.84 Patentblatt 84/26**

(84) Benannte Vertragsstaaten: **AT BE CH DE FR GB IT LI NL SE**

(56) Entgegenhaltungen:
**EP - A - 0 041 180**
**US - A - 4 158 608**

(73) Patentinhaber: **BAYER AG, Konzernverwaltung RP Patentabteilung, D-5090 Leverkusen 1 Bayerwerk (DE)**

(72) Erfinder: **Metzger, Karl Georg, Dr., Pahlkestrasse 15, D-5600 Wuppertal 1 (DE)**
Erfinder: **Pfitzner, Jörg, Dr., Claudiusweg 3, D-5600 Wuppertal 1 (DE)**
Erfinder: **Schmidt, Delf, Dr., Am Eckbusch 55b, D-5600 Wuppertal 1 (DE)**
Erfinder: **Weyland, Horst, Dr., Fichtestrasse 12, D-2850 Bremerhaven (DE)**
Erfinder: **Benz, Günter, Dr., Am Bölkumer Busch 5, D-5620 Velbert 15 (DE)**
Erfinder: **Schröder, Theo, Dr., Gellertweg 38, D-5600 Wuppertal 1 (DE)**

**EP 0 057 349 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft die neue Verbindung Komponente B (Komponente A, vgl. EP-A-0 057 812), ein mikrobiologisches Verfahren zu ihrer Herstellung aus einem Streptomyces-Stamm sowie ihre Verwendung als Arzneimittel, insbesondere als antimikrobielles Mittel in der Human- und Tiermedizin.

Es ist bereits bekanntgeworden, daß eine Reihe von Verbindungen mikrobieller Herkunft antimikrobielle Wirkungen besitzen. Diese Antibiotika sind teilweise in ihren Wirkungsspektren nicht voll befriedigend. Sie weisen häufig noch weitere Nachteile auf. β-Lactamantibiotika werden oft durch Penicillinase inaktiviert, Chloramphenicol, Tetracycline und Streptomycin zeigen in vielen Fällen erhebliche unerwünschte Nebenwirkungen (vgl. Walter, Heilmeyer, Antibiotika Fibel, Georg Thieme Verlag, Stuttgart, 3. Auflage, 1969, Seiten 248, 278—280 und 311—319).

Es wurde nun gefunden, daß man eine neue antibiotisch wirksame Verbindung erhält, wenn man den Stamm Streptomyces spec. WS 116 in einem Nährmedium züchtet und die Verbindung aus dem Nährmedium isoliert.

Die neue Verbindung weist starke antimikrobielle Wirkung auf. Sie kann eisenfrei oder eisenhaltig sein. Beide Formen können erfolgreich als antibakterielles Mittel eingesetzt werden.

Die neue Verbindung in der eisenfreien Form wird durch folgende Eigenschaften charakterisiert:

1. Die Elementaranalyse C 42,3%; H 6,2%; N 15,7%; O 31,4%; S 3,6%.
   Es muß hier darauf hingewiesen werden, daß bei höhermolekularen Naturstoffen die Fehlerbreite der Elementaranalyse größer sein kann, als allgemein üblich, daher ist eine genaue Bestimmung der Summenformel oft nicht möglich (R. B. Woodward, Angew. Chem. 69, S. 50—51 [1957]).
2. Die gefriergetrocknete Verbindung zersetzt sich bei etwa 185° C.
3. Ultraviolettabsorptionsspektrum:
   Das UV-Spektrum wurde an einer wäßrigen Lösung der Verbindung (c = 1,786 mg in 25 ml $H_2O$) aufgenommen. Die Spektren in saurer (bzw. basischer Lösung) wurden an einer Lösung gemessen, die durch Zusatz von 100 Mikrolitern 1 n-Salzsäure (bzw. Natronlauge) zu 3 ml der obigen Lösung hergestellt wurde.

Tabelle 1

Maxima ($\lambda$ max) und Extinktionen [$E^{1\%}_{cm}$] der Verbindung

| | $\lambda$ max [nm] | [$E^{1\%}_{cm}$] |
|---|---|---|
| neutral | 282 | 123 |
| sauer | 304 | 108 |
| basisch | 277 | 118 |

4. Das IR-Absorptionsspektrum der Verbindung wird in Figur 1 wiedergegeben. (Abszisse: Wellenzahl in $cm^{-1}$, Ordinate: Absorption)
   Es zeigt, wenn die Substanz zu KBr Preßlingen verpreßt wird, bei folgenden Wellenlängen (ausgedrückt in $cm^{-1}$) Absorptionsbanden:

Tabelle 2

| Wellenlänge in cm 1 | Wellenlänge in cm 1 |
|---|---|
| 3388 | 1390 |
| 2944 | 1299 |
| 1695 | 1240 |
| 1648 | 1070 |
| 1545 | 1050 |
| 1457 | 975 |
| 1417 | |

5. Das $^1H$-Kernresonanzspektrum gibt die Signallage in Teilen pro Million (ppm) und Schwingungen pro Sekunde gemäß Figur 2 an. Es wurde an einer wäßrigen Lösung der Verbindung mit TMS-Na-Salz als Standard (extern) an einem WH-360-Spektrometer der Firma Bruker bei einer Feldstärke von 360 MHz aufgenommen.
6. Das 13-C-Kernresonanzspektrum wurde an einem WM-250-Spektrometer der Fa. Bruker bei einer Feldstärke von 62,71 MHz an einer wäßrigen Lösung der Verbindung aufgenommen, wobei auf Dioxan als externen Standard (Shiftlage 67.400 ppm rel. zu TMS = 0) umgerechnet wurde.
Das 13-C-Kernresonanzspektrum gemäß Figur 3 zeigt die folgenden Signale angegeben in Teilen pro Million (ppm) und Schwingungen pro Sekunde (Hz) in ihren relativen Intensitäten an:

Tabelle 3

Shiftlagen und Intensitäten der Signale im 13-C-Kernresonanzspektrum bezogen auf Dioxan = 67.400 ppm (extern)

| Signal NS | rel. Intensität | Signallage (ppm) |
|---|---|---|
| 1 | 3.344 | 175.878 |
| 2 | 2.094 | 174.610 |
| 3 | 1.628 | 174.289 |
| 4 | 1.634 | 174.032 |
| 5 | 1.708 | 171.786 |
| 6 | 1.25 | 167.843 |
| 7 | 1.966 | 156.220 |
| 8 | 2.580 | 153.171 |
| 9 | 3.806 | 138.745 |
| 10 | 3.979 | 98.082 |
| 11 | 3.729 | 80.173 |
| 12 | 3.207 | 76.001 |
| 13 | 3.044 | 69.984 |

Fortsetzung

| Signal NS | rel. Intensität | Signallage (ppm) |
|---|---|---|
| 14 | 83.487 | 67.400 |
| 15 | 2.996 | 64.094 |
| 16 | 4.082 | 62.072 |
| 17 | 1.751 | 58.606 |
| 18 | 2.995 | 56.392 |
| 19 | 4.129 | 54.418 |
| 20 | 4.430 | 54.322 |
| 21 | 2.449 | 54.017 |
| 22 | 2.983 | 51.064 |
| 23 | 3.998 | 48.111 |
| 24 | 2.442 | 29.834 |
| 25 | 4.590 | 29.048 |
| 26 | 5.047 | 28.967 |
| 27 | 2.559 | 23.319 |
| 28 | 2.276 | 23.223 |
| 29 | 1.510 | 22.634 |
| 30 | 7.743 | 20.222 |

7. Der optische Drehwert beträgt sofort nach dem Lösen $[\alpha]_D^{20}$ = $-27{,}725°$ (c=0,2723% in Wasser).
8. Die Verbindung ist unbeschränkt in Wasser löslich, löst sich etwas in Methanol, DMF und DMSO und ist schwerlöslich in Chloroform, Ether, Essigester und Petrolether.
9. Die Verbindung ist ein farbloser, amorpher Feststoff, dessen wäßrige Lösung neutral reagiert.
10. Die $R_f$-Werte der Verbindung in der eisenfreien und eisenhaltigen Form im Vergleich mit anderen Verbindungen in verschiedenen Laufmitteln gibt Tabelle 4.
    a) DC-Fertigplatten Kieselgel 60 F 254 (Merck) Anfärbung: 1. Ninhydrin; 2. 5% $FeCl_3 \times 6\,H_2O$ in 0,5 n-HCl
    Fließmittel (FM 1): Isobutanol/Ethanol/Ammoniak = 9/1/5 (Volumenteile)
    Fließmittel 2 (FM 2): Isobutanol/Ethanol/Ammoniak = 4/1/5 (Volumenteile)
    10 cm Laufstrecke/Auftrag 50 µg in dest. Wasser

Tabelle 4a

| Substanz | FM 1 | FM 2 |
|---|---|---|
| Neomycinsulfat | 0,01 | 0,16 |
| 2-Desoxystreptamin × 2 HCl | 0,03 | 0,19 |
| Sisomicin Base | 0,14 | 0,42 |
| erfindungsgemäße Verbindung, eisenfrei | 0,02 | 0,25 |
| dto., eisenhaltig | 0 | 0,22 |

b) DC-Fertigplatten Cellulose F (Merck)
Anfärbung: 1. Ninhydrin; 2. 5% $FeCl_3 \times 6$ HCl
Fließmittel 1 (FM 1): 1-Butanol/Eisessig/dest. Wasser = 4/1/5
Fließmittel 2 (FM 2): 1-Butanol/Eisessig/dest. Wasser = 4/1/2
Fließmittel 3 (FM 3): 1-Propanol/Pyridin/Eisessig/dest. Wasser = 15/10/3/12
10 cm Laufstrecke/Auftrag 50 µg in dest. Wasser

Tabelle 4b

| Substanz | FM 1 | FM 2 | FM 3 |
|---|---|---|---|
| Neomycinsulfat | 0,06 | 0,06 | 0,06 |
| 2-Desoxystreptamine $\times$ 2 $H_2O$ | 0,10 | 0,13 | 0,42 |
| Sisomicin Base | 0,16 | 0,27 | 0,49 |
| erfindungsgemäße Verbindung, eisenfrei | 0,27 | 0,33 | 0,80 |
| dto., eisenhaltig | 0,15 | 0,19 | 0,71 |

11. Die neue Verbindung läßt sich in ihrer eisenfreien Form auf der Dünnschichtplatte mit $FeCl_3$*), alk. Kaliumpermanganat, Jod, Ninhydrin und im UV-Licht bei 254 oder 280 nm durch Fluoreszenzlöschung sichtbar machen.
12. Die Totalhydrolyse der Verbindung mit wäßriger 57%iger Jodwasserstoffsäure zeigt bei der Aminosäureanalyse, wenn im verschlossenen Glas bei 110°C 24 Stunden hydrolysiert wird, daß die Substanz drei Einheiten Ornithin neben einer Einheit Serin enthält. Daneben wurden nur Spuren anderer natürlicher Aminosäuren gefunden.
Durch Derivatisierung, Isolierung von Hydrolyseprodukten und deren Derivaten konnte die Konstitution der erfindungsgemäßen Verbindung Komponente B aufgelöst werden. Danach hat Komponente B folgende Konstitution:

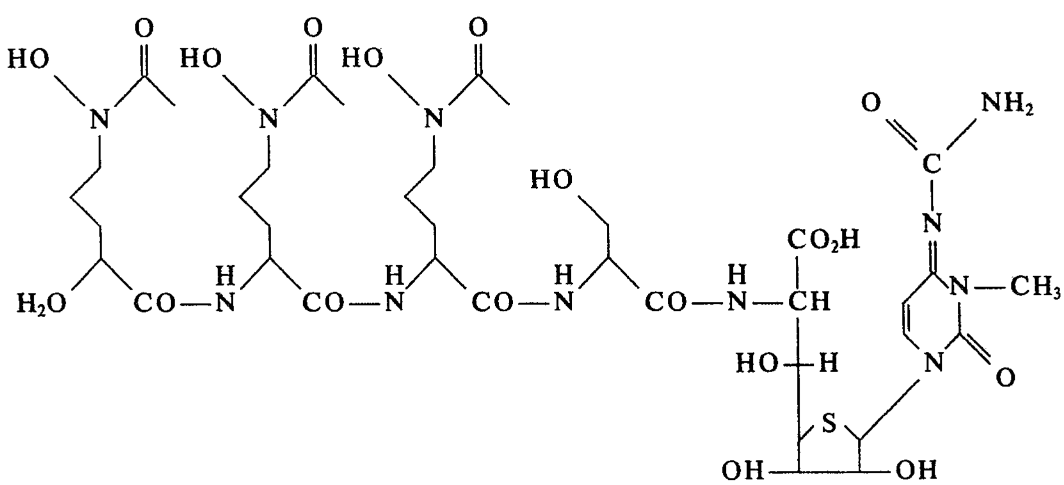

Die erfindungsgemäße Verbindung wird durch submerse Kultur eines Streptomyceten-Stammes in geeigneten Nährlösungen unter geeigneten physikalischen Bedingungen erzeugt. Sie wird aus der Kulturlösung durch Extraktion oder durch Adsorption abgetrennt und durch weitere geeignete Methoden angereichert.

Für das Herstellungsverfahren kann der neue Stamm Streptomyces spec. WS 116 aus der Ordnung der Actinomycetales, Familie Streptomycetaceae, Gattung Streptomyces oder von ihm abstammende Varianten und Mutanten eingesetzt werden. Dieser Stamm wurde aus einer marinen Bodensedimentprobe der Ibero-Canarischen See isoliert. Er wurde unter der Nummer DSM 1692 am 7. 12. 1979 bei der Deutschen Sammlung für Mikroorganismen, Göttingen hinterlegt.

*) Die Sprühreagentien wurden nach den üblichen Vorschriften (z. B. E. Stahl, Dünnschichtchromatographie, 2. Auflage, Springer Verlag, Berlin) hergestellt.

a) Die Sporen sind ellipsoid. Sie haben die Größe von 0,4—0,7 × 0,7—1,2 µm und eine glatte Oberfläche.
b) Die Farbe des Luftmycels ist zu Anfang kreideweiß, im ausgereiften Zustand gelblich (Griseus-Typ).
c) Die Sporenketten sind gerade oder gewellt (Rectus-Flexibilis-Typ) und monopodial verzweigt.
d) Auf Pepton-Eisen-Agar sowie auf Tyrosin-Agar wurde kein schwarzbraunes Pigment gebildet. Der Stamm ist nicht chromogen.

Die zusammengefaßten Bestimmungsmerkmale identifizieren den Stamm WS-116 als zur Art Streptomyces griseus Waksman et Henrici gehörend.

Für das Verfahren zur Herstellung der erfindungsgemäßen Verbindung verwendet man Nährmedien, die die üblichen Kohlenstoff- und Stickstoffquellen und die notwendigen Salze enthalten. Als Kohlenstoffquelle können verwendet werden: Kohlehydrate, insbesondere Polysaccharide, wie Stärke oder Dextrine, Disaccharide wie Maltose oder Rohrzucker, Monosaccharide, wie Glucose oder Xylose, Zuckeralkohole, wie Mannit oder Glycerin, Carbonsäuren, wie Citronensäure, Äpfelsäure, Essigsäure oder deren Gemische, außerdem auch natürlich vorkommende Gemische wie Malzextrakte. Überraschenderweise wurden mit Carbonsäuren, insbesondere mit Citronensäure als Haupt-C-Quelle die höchsten Wirkstoffausbeuten erzielt. Als Stickstoffquelle können die üblichen Stickstoffquellen Verwendung finden, so z. B. Eiweißstoffe, Eiweißhydrolysate, Aminosäuren wie Glutaminsäure, Asparaginsäure, Arginin, Lysin, Ornithin oder Serin, außerdem Nucleosidbasen wie Cytosin oder Uracil, Ammonium-Salze, Nitrate, natürlich vorkommende komplexe Stoffe, wie Peptone, Maisquellwasser, Sojabohnenmehl, Fleischextrakte, Hefeextrakte und geeignete Mischungen derselben. Besonders hohe Wirkstoffausbeuten erhält man, wenn zu den üblichen komplexen N-Quellen L-Ornithin und L-Serin im Verhältnis 3 : 1, z. B. 0,3% und 0,1%, in sterilfiltrierter Form dem Medium zugesetzt werden.

Als Hilfsstoffe werden im Nährmedium Mineralsalze benötigt, z. B. Phosphate, Sulfate, Carbonate, Nitrate oder Chloride des Natriums, Kaliums, Calciums, Magnesiums, Eisens, Zinks, Kupfers, Molybdäns, Kobalts, Nickels und Mangans. Als bedeutsam erwies sich die Anwesenheit von etwa 0,01% $FeCl_3$. Zum Teil sind die Mineralsalze, auch das $FeCl_3$, in den notwendigen Konzentrationen in den obengenannten Kohlenstoff- oder Stickstoffquellen oder im verwendeten Wasser als Bestandteile enthalten.

Weiterhin können als Hilfsstoffe noch Antischaummittel der verschiedensten Art Verwendung finden, wie z. B. Sojaöl, Polyole oder Silikone.

Als wichtigstes Verdünnungsmittel für die Nährmedien ist Wasser zu nennen.

Bei der Durchführung des Herstellungsverfahrens wird unter aeroben bzw. mikroaerophilen Bedingungen gearbeitet; die Kultur kann gemäß üblicher Methoden, so z. B. unter Verwendung von Schüttelkulturen oder von belüfteten Fermenterkulturen, im üblichen Batch- oder Fedbatch-Verfahren durchgeführt werden. Die Prozentverhältnisse (jeweils Gewichtsprozente) der Nährlösungsbestandteile können in weiten Bereichen schwanken, im allgemeinen machen die Kohlenstoffquellen insgesamt 0,5—8%, vorzugsweise 0,6—6% aus, die Stickstoffquellen insgesamt 0,1 bis 4%, vorzugsweise 0,5 bis 2% aus; die Salze liegen in üblichen Konzentrationen vor, vorzugsweise im Bereich zwischen 0,001 bis 0,5 Gew.-%. Die Antischaummittel liegen in 0- bis 1%iger Konzentration vor. Die zur Sterilisation angewandten Temperaturen liegen bei 100 bis 140°C, vorzugsweise bei 120 bis 130°C, empfindliche Substanzen wie Aminosäuren werden sterilfiltriert.

Die pH-Werte der wachsenden Kulturen liegen zwischen 5 und 10, vorzugsweise zwischen 6 und 9,5. Die Züchtungstemperatur kann zwischen 15 und 35°C, vorzugsweise zwischen 20 und 30°C liegen. Es hat sich herausgestellt, daß die Menge des sich in der Kulturbrühe anreichernden Antibioticums im allgemeinen ihr Maximum etwa 1 bis 10, vorzugsweise etwa 2 bis 6 Tage nach Züchtungsbeginn erreicht. Der Endpunkt der Fermentation wird mit Hilfe von biologischen Tests ermittelt. (Wirkung gegen E. coli in einem üblichen Agardiffusionstest.)

Die erfindungsgemäße Verbindung wird aus dem Kulturfiltrat durch Extraktion mit Mischungen aus Phenol/Chloroform oder aber durch Adsorption an Aktivkohle bzw. an geeignete Harze isoliert. Vorteilhaft ist die Bindung der erfindungsgemäßen Verbindung an unspezifische Adsorberharze auf Polystyrolbasis (z. B. Amberlite® XAD der Fa. Rohm & Haas oder Lewatit® OC 1031 der Fa. Bayer). Überraschenderweise wurde gefunden, daß die erfindungsgemäße Verbindung besonders fest von derartigen Harzen gebunden wird, wenn man vor dem Adsorptionsvorgang Eisensalz insbesondere Eisenchlorid in Konzentration von 0,05 bis 0,2, insbesondere ca. 0,1 g/Liter Kulturbrühe, hinzugibt. Die Adsorption führt man im pH-Bereich 3—9, insbesondere im Bereich 5—7 durch. Die Desorption der erfindungsgemäßen Verbindung wird fraktioniert durch Mischungen aus Wasser und organischen Lösungsmitteln, insbesondere Wasser/Methanol, vorgenommen. Die aktiven Fraktionen werden vereinigt, auf ein kleines Volumen eingeengt und lyophilisiert. Man erhält ein 0,5—3%iges Rohprodukt, daß die erfindungsgemäße Verbindung neben anderen Substanzen enthält.

Ausgehend von diesem Rohprodukt kann die weitere Anreicherung der erfindungsgemäßen Verbindung durch eine Kombination von Anionen- (z. B. DEAE-Sephadex® A 25, Fa. Pharmacia) bzw. Kationen-Austauschchromatographie (z. B. SP- oder CM-Sephadex® C 25, Fa. Pharmacia) durchgeführt

werden. Man erhält dadurch ein 30—50%iges Präparat, da begleitende Peptide nicht abgetrennt werden. Die Abtrennung dieser Peptide und damit die Darstellung der reinen Verbindung gelingt durch Adsorptions- bzw. Verteilungschromatografie der ca. 30—50%igen Verbindung an Kieselgel im System Isobutanol/Ethanol/25% konz. Ammoniak = 9/1/5 (Volumenteile).

Dieses Trennverfahren ist jedoch mit Substanzverlusten verbunden.

Wesentlich einfacher gelingt die Trennung durch Affinitätschromatografie an einer $Fe^{+++}$-haltigen Säule. Dazu überführt man einen Kationenaustauscher auf Polystyrol oder -acrylatharzbasis (z. B. Dowex® 50 WX 4, Fa. DOW Chemical) oder auf Polydextranbasis (z. B. Sephadex® C 25, Fa. Pharmacia) mit $FeCl_3$-Lösung in die $Fe^{+++}$-Form. Auf das Harz in der $Fe^{+++}$-Form wird nun die Lösung des Rohproduktes aufgetragen und anschließend mit Wasser nachgewaschen. Dann eluiert man mit einem Puffer hoher Ionenstärke, z. B. 0,2 m-$NaH_2PO_4$/0,3 m-NaCl. Dieser Puffer eluiert die Hauptmenge der inaktiven Begleitpeptide. Anschließend eluiert man die wirksamen Substanzen von der Säule mit dem gleichen Puffer, jedoch unter Zusatz von 0,05 m-Ethylendiamintetraessigsäure oder einem anderen Eisenkomplexbildner (z. B. Citrat). Die aktiven Fraktionen werden vereinigt und über eine Säule mit unspezifischem Adsorptionsharz (z. B. Lewatit® OC 1031, Fa. Bayer) gegeben, wobei die wirksamen Substanzen gebunden werden. Man eluiert mit Methanol, engt ein und lyophilisiert.

Die erfindungsgemäße Verbindung wird durch Verteilungschromatographie an Sephadex® G 25 in nBuOH/iso BuOH/0,2 m $(NH_4)_2SO_4$ = 2/1/1 rein erhalten. Die Reindarstellung des erfindungsgemäßen Produktes wird ferner durch einfache Chromatographie des Wirkstoffgemisches an einer CM-Cellulose-Säule in der $H^+$-Form mit dest. Wasser ohne irgendwelche Zusätze durchgeführt. Das aktive Eluat, in Fraktionen aufgefangen, wird lyophilisiert.

Die gute antimikrobielle Wirksamkeit der erfindungsgemäßen Verbindung wird durch in vitro- und in vivo-Versuche demonstriert.

In vitro wird im Agar-Verdünnungstest nach dem international üblichen Test (American National Committee for Clinical Laboratory Standards = NCCLS) Wirksamkeit gegen Enterobacteriaceae, z. B. E. coli 14 und E. coli C 165 gefunden.

In vivo wird an der weißen Maus eine $ED_{100}$ (vollständige Heilung) bei Infizierung mit E. coli Neumann bei 3 mg Antibiotikum/kg Körpergewicht gefunden; Therapie: einmalig 30 Minuten nach Infektion subcutan.

Die neue Verbindung Komponente B kann in üblichen pharmazeutischen Zusammensetzungen angewendet werden. Die pharmazeutischen Zubereitungen enthalten den erfindungsgemäßen Wirkstoff und übliche, geeignete, nicht-toxische Träger- und Hilfsstoffe.

Geeignete Zubereitungen und die dazu notwendigen Hilfs- und Trägerstoffe sind beispielsweise aus der deutschen Offenlegungsschrift 25 10 161 bekannt.

Komponente B (eisenhaltig)

| KEIM | MHK in µg/ml |
|---|---|
| Escherichia coli T7 | ≤ 0,2 |
| Escherichia coli F14 | ≤ 0,1 |
| Escherichia coli N | 0,2 |
| Klebsiella 57 US | ≤ 0,1 |
| Klebsiella 63 | ≤ 0,1 |
| Staphylococcus aureus 133 | ≤ 0,2 |

Die Bestimmung der minimalen Hemmkonzentration (MHK) erfolgte mit einem Inoculator im Agarverdünnungsverfahren mit einer Einsaatdichte von $10^4$ pro Impf-Punkt. Die MHK ist die Konzentration, bei der keine Bakterienkolonien wachsen.

Komponente B (eisenhaltig)/Tierversuch (Mäuse)

| Infektionskeim | $ED_{100}$ nach intraperitonealer Infektion und subcutaner Behandlung in mg/kg |
|---|---|
| E. coli Neumann | 0,5 |
| E. coli 205 Kn | 0,1 |
| Klebsiella pneumoniae 63 | 0,2 |
| Staphylococcus aureus 133 | 0,6 |

Die Tiere waren mit einer Bakterienmenge infiziert, welche innerhalb von 24 Std. zum Tod der Kontrolltiere führte. Die $ED_{100}$ ist die Dosis, die alle infizierten und behandelten Tiere überleben läßt.

## Beispiel 1a

Die Nährlösung in der der Produktionsstamm Streptomyces spec. WS 116 in den Vorkulturen kultiviert wird, setzt sich aus 1 Gew.-% Glucose, 1,3% Hefeextrakt, 0,05% Polyol und Leitungswasser zusammen. Der pH wird vor dem Sterilisieren auf 7,0 eingestellt. 4 × 1000-ml-Erlenmeyerkolben, die je 150 ml dieser Nährlösung enthalten, werden mit dem Produktionsstamm beimpft und 4 Tage bei 28°C auf einer Rundschüttelmaschine bei 220 Umdrehungen/min inkubiert. Mit diesen Vorkulturen wird eine zweite Vorkultur, ein Labor-Fermenter, der 20 l der oben erwähnten Nährlösung enthält, beimpft, bei 200 Umdrehungen/min, 10 l Luft/min und 28°C 2 Tage inkubiert. Mit 20 l dieser Kultur wird ein Produktionsfermenter beimpft, der 600 l Nährlösung mit folgender Zusammensetzung enthält: 0,7 Gew.-% Citronensäure, 0,8% Hefeextrakt, 0,2% entfettetes Sojabohnenmehl, 0,2% Maisquellwasser und 0,05% Silicon in Leitungswasser. Der pH dieser Nährlösung wird mit Kalilauge auf 6,4 vor der Sterilisation eingestellt. Die Inkubation der Produktionskultur erfolgt über 2—4 Tage bei 26°C bei einer Rührung von 50 Umdrehungen/min und einer Belüftung von nur 90 l Luft/min. Die Fermentation wird bei optimaler antibiotischer Hemmaktivität der Kultur abgestoppt.

## Beispiel 1b

2 × 150 ml Vorkultur werden wie im Beispiel 1 angegeben, angezogen. Mit diesen Vorkulturen wird ein 10-l-Produktionsfermenter beimpft, dessen Nährlösung, in Leitungswasser angesetzt, folgende Zusammensetzung enthält:

0,7 Gew.-% Citronensäure,
0,8 Gew.-% Hefeextrakt,
0,2 Gew.-% entfettetes Sojabohnenmehl,
0,2 Gew.-% Maisquellwasser,
0,3 Gew.-% L-Ornithin,
0,1 Gew.-% L-Serin und
0,05 Gew.-% Silicon.

Alle Bestandteile außer Ornithin und Serin werden wie üblich im Kulturgefäß sterilisiert. Es wird ein pH-Wert von 6,4 vor der Sterilisation eingestellt. Ornithin und Serin werden in $H_2O$ dest. gelöst dem Ansatz sterilfiltriert zugesetzt.

Die Inkubation der Produktionskultur erfolgt über 2—4 Tage bei 26°C mit einer Rührung von 200 Umdrehungen/min und einer Belüftung von nur 1,5 l Luft/min. Die Fermentation wird bei optimaler antibiotischer Hemmaktivität des Kulturüberstandes abgestoppt.

## Beispiel 2

4000 l Kulturbrühe (pH = 9,06) wurden mit 50 l 1 : 1 verdünnter HCl auf pH 6,2 eingestellt. Man setzte 400 g $FeCl_3 \cdot 6\ H_2O$ hinzu, rührte und gab dann 25 l verdünnte NaOH bis pH 7 unter Rühren hinzu. Dann wurde mit 200—250 l/h im Westfalia-Separator separiert. Der Überstand wurde durch eine 30 × 70 cm mit Lewatit® OC 1031 (= unsp. Adsorptionsharz der BAYER AG) gefüllte Säule gegeben, der Durchlauf kanalisiert, da inaktiv. Die Säule wurde mit 1000 l entionisiertem Wasser gewaschen, das Waschwasser war inaktiv und wurde verworfen. Die Säule wurde nun mit 1000 l 15% Methanol gewaschen, dieses inaktive Waschwasser wurde ebenfalls verworfen. Man eluierte die Aktivität nun mit 50% Methanol von der Säule und sammelte 100-l-Fraktionen. Die aktiven Eluate 2 und 3 wurden vereinigt, eingeengt im Dünnschichtverdampfer auf ca. 20 l und dann lyophilisiert. Man erhielt 342 g Rohprodukt der erfindungsgemäßen Verbindung mit einem Gehalt von ca. 2,5% (Komponenten A und B; Komponente A vgl. EP-A-0 057 812).

Beispiel 3

Man löste die obige Rohsubstanz mit 6 Litern $H_2O$ und versetzte unter Rühren mit 25 ml 50%iger $FeCl_3$-Lösung. Der sich bildende Niederschlag wurde nach 15 min Rühren abzentrifugiert (Hettich Rota Magna Zentrifuge, 1,5 l Becher, 30 min, 4000 UpM). Der Überstand wurde mit Schwerkraft über eine 8 x 45 cm hoch mit SP-Sephadex® C 25 $Fe^{III}$ gefüllte Säule gegeben. Die Fließrate betrug 4 l pro Stunde. Man wusch die schwarz gefärbte Säule mit 5 l dest. $H_2O$ nach und schloß dann 10 l 0,2 m $NaH_2PO_4$/0,3 NaCl Puffer an. Durchlauf und Waschwasser enthielten weniger als 5% der eingesetzten antibiotischen Aktivität. Die jetzt nur noch hellbraun gefärbte Säule wurde nun mit 0,2 m $NaH_2PO_4$/0,3 m NaCl/0,05 m AeDTA eluiert (Fließrate 2—3 l/h), das Säuleneluat fraktioniert in 500-ml-Portionen aufgefangen. Die aktiven Fraktionen 6—14 wurden vereinigt und über eine 5 x 10 mit Lewatit® OC 1031 gefüllte Säule gegeben. Die Fließrate betrug 3 l/h. Man wusch anschließend mit destilliertem Wasser bis mit $AgNO_3$ keine $Cl^-$ im Säuleneluat nachzuweisen war (ca. 6 Liter, Fließrate 5 l/h). Anschließend eluierte man die Säule mit 3 Liter 90% Methanol, die im batch aufgefangen, eingeengt und lyophilisiert wurden. Ausbeute: 6,74 g, 82,6% Komponente A und B.

Beispiel 4

Die halbe Ausbeute aus Beispiel 2 = 3,37 g wurden in 100 ml dest. $H_2O$ gelöst. Die Leitfähigkeit betrug 210 µS.

Die Lösung wurde auf eine 5 x 30 cm mit CM-Cellulose in der $H^+$-Form (CM-Cellulose C 52, Fa. Whatmann) gefüllte Säule aufgetragen. Man entwickelte mit dest. Wasser bei einer Fließrate von 840 ml/H. Das Eluat wurde an Hand der Refrakraktions-, Leitfähigkeits- und Extraktionskurve geschnitten. Man erhielt Vorlauf (inaktiv) 980 mg

| | |
|---|---|
| Fraktion 1 | 427 mg |
| Fraktion 2 | 674 mg |
| Fraktion 3 | 363 mg |
| Fraktion 4—6 | 475 mg |
| Fraktion 7 | 253 mg |
| Fraktion 8 | 96 mg |

Fraktionen 2—8 zusammengefaßt sind die erfindungsgemäße Verbindung Komponente B.

**Patentansprüche**

1. Verbindung der allgemeinen Formel

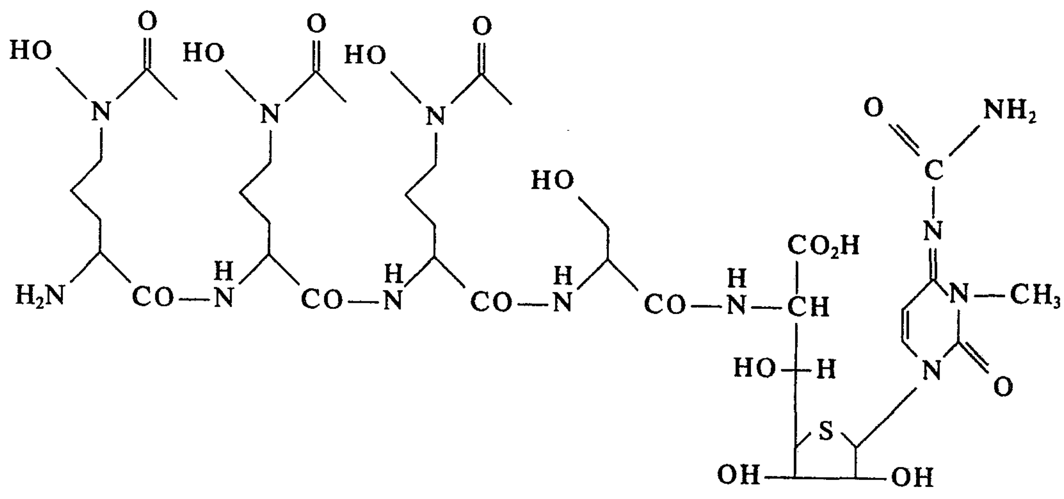

und deren Eisen-III-Komplex.

2. Verbindung mit einem Zersetzungspunkt von 185°C, einer optischen Drehung $[\alpha]_D^{20} = -27{,}7°$ (c=0,2723 in Wasser, sofort nach dem Lösen gemessen), das in Wasser bei pH 7 leicht, in Methanol, Dimethylformamid und Dimethylsulfoxid merklich und in Chloroform, Ether, Essigester und Petrolether schwer löslich ist, im UV-Spektrum in Wasser (sauer) ein Maximum bei 304 nm, als KBr-Preßling im infraroten Bereich Absorptionsmaxima bei 3388, 2944, 1695, 1648, 1545, 1457, 1417, 1390, 1299, 1240, 1070, 1050 und 975 aufweist und bei saurer Hydrolyse Ornithin und Serin im Verhältnis 3 : 1 freisetzt, und deren Eisen-III-Komplex.

3. Antibiotikum gemäß einer der Ansprüche 1 oder 2, gekennzeichnet durch das 1H-NMR-Spektrum gemäß Fig. 2, durch 13C-NMR-Spektrum gemäß Fig. 3 und durch ein Maximum bei 282 nm im UV-Spektrum in Wasser bei pH 7.

4. Verfahren zur Herstellung einer eisenhaltigen oder eisenfreien Verbindung gemäß einem der Ansprüche 1—3, dadurch gekennzeichnet, daß man unter submersen, aeroben Bedingungen Streptomyces spec. WS 116 (DSM 1692) in einem assimilierbaren Kohlenstoff, Stickstoff sowie Mineralsalze, insbesondere Eisensalze enthaltenden Nährmedium bei einer Temperatur von 15 bis 35°C züchtet und die gebildete Verbindung aus der Fermentationsflüssigkeit isoliert.

5. Verfahren nach Anspruch 4, dadurch gekennzeichnet, daß man zur Isolierung die erfindungsgemäße Verbindung an ein unspezifisches Adsorptionsharz bindet und mit Mischungen aus Wasser und organischem Lösungsmittel desorbiert.

6. Verfahren nach den Ansprüchen 4—5, dadurch gekennzeichnet, daß man die Kulturbrühe vor der Adsorption mit einem Eisensalz versetzt.

7. Verfahren nach den Ansprüchen 4—6, dadurch gekennzeichnet, daß man zur Isolierung das rohe Antibiotikum über einen Kationenaustauscher in der $Fe^{3+}$-Form chromatographiert, inaktive Begleitstoffe mit einem Puffer hoher Ionenstärke eluiert und das Antibiotikum mit dem gleichen Puffer und Zusatz eines Eisen-Komplexbildners eluiert.

8. Verfahren nach Anspruch 7, dadurch gekennzeichnet, daß man das nach Anspruch 7 erhaltene Produkt über eine CM-Cellulose-Säule in der $H^+$-Form chromatographiert und mit Wasser fraktioniert eluiert.

9. Arzneimittel mit einem Gehalt an der Verbindung gemäß einem der Ansprüche 1—3.

10. Verbindung gemäß einem der Ansprüche 1—3 zur Verwendung bei der Bekämpfung bakterieller Infektionen.

11. Verfahren nach den Ansprüchen 4—8, dadurch gekennzeichnet, daß in einer Nährlösung fermentiert wird, die als Hauptkohlenstoffquelle Citronensäure, mehrere komplexe Stickstoffquellen (Hefeextrakt, Sojabohnenmehl, Maisquellwasser) und die Aminosäuren L-Ornithin und L-Serin enthält.

12. Verfahren nach den Ansprüchen 4 bis 8, dadurch gekennzeichnet, daß in einer voll synthetischen Nährlösung fermentiert wird, die neben Mineralsalzen als Haupt-C-Quelle Citronensäure und als einzige Stickstoffquelle L-Arginin enthält.

13. Verfahren nach den Ansprüchen 4 bis 8, dadurch gekennzeichnet, daß zu einer Nährlösung zur Ausbeutesteigerung ein Gemisch von Citronensäure-L-Arginin und Salzen in solchen Raten nachgefüttert wird, daß ein konstanter pH-Wert aufrechterhalten wird.

## Claims

1. A compound of the general formula

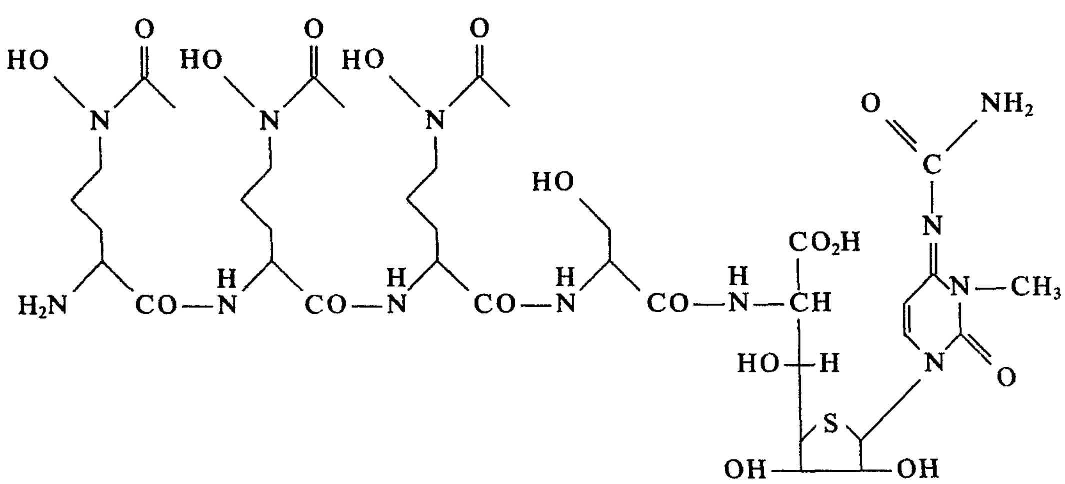

and the iron (III) thereof.

2. A compound with a decomposition point of 185°C and an optical rotation $[\alpha]_D^{20} = -27,7°$ (c = 0.2723 in water, measured immediately after the substance has dissolved), which is readily soluble in water at pH 7, noticeably soluble in methanol, dimethylformamide and dimethylsulphoxide and sparingly soluble in chloroform, ether, ethyl acetate and petroleum ether, has a maximum at 304 nm in the UV spectrum in water (acid), as a KBr pressed plate has absorption maxima at 3388, 2944, 1695, 1648, 1545, 1457, 1417, 1390, 1299, 1240, 1070, 1050 and 975 in the infrared region, and releases ornithine and serine in the ratio 3 : 1 on acid hydrolysis, and the iron (III) complex thereof.

3. An antibiotic according to one of Claims 1 or 2, characterised by the 1H-NMR spectrum according to Figure 2, by the 13C-NMR spectrum according to Figure 3 and by a maximum at 282 nm in the UV spectrum in water at pH 7.

4. Process for the preparation of an iron-containing or iron-free compound according to one of the Claims 1—3, characterised in that Streptomyces spec. WS 116 (DSM 1692) is grown, under submerse, aerobic conditions, in a nutrient medium containing assimilable carbon, nitrogen and mineral salts, particularly iron salts, at a temperature of from 15 to 35°C, and the compound formed is isolated from the fermentation liquid.

5. Process according to Claim 4, characterised in that the compound according to the invention is isolated by binding it to a non-specific adsorption resin and desorbing it with mixtures composed of water and an organic solvent.

6. Process according to Claims 4—5, characterised in that an iron salt is added, before the adsorption, to the culture broth.

7. Process according to Claims 4—6, characterised in that the crude antibiotic is isolated by subjecting it to chromatography over a cation exchanger in the $Fe^{3+}$ form, eluting inactive accompanying substances with a buffer of high ionic strength and eluting the antibiotic with the same buffer and with the addition of an iron complexforming agent.

8. Process according to Claim 7, characterised in that the product obtained according to Claim 7 is subjected to chromatography over a CM-cellulose column in the $H^+$ form and is fractionally eluted with water.

9. A medicament having a content of the compound according to one of the Claims 1—3.

10. A compound according to one of Claims 1—3 for use in combating bacterial infections.

11. Process according to Claims 4—8, characterised in that the fermentation is carried out in a nutrient solution which contains citric acid as the main carbon source, several complex nitrogen sources (yeast extract, soya bean flour and corn-steep liquid) and the aminoacids L-ornithine and L-serine.

12. Process according to Claims 4 to 8, characterised in that the fermentation is carried out in a completely synthetic nutrient solution which contains in addition to mineral salts citric acid as the main C source and L-arginine as the only nitrogen source.

13. Process according to Claims 4 to 8, characterised in that in order to increase the yield a mixture of citric acid, L-arginine and salts is further fed to a nutrient solution at such rates that a constant pH value is maintained.

**Revendications**

1. Composé de formule générale:

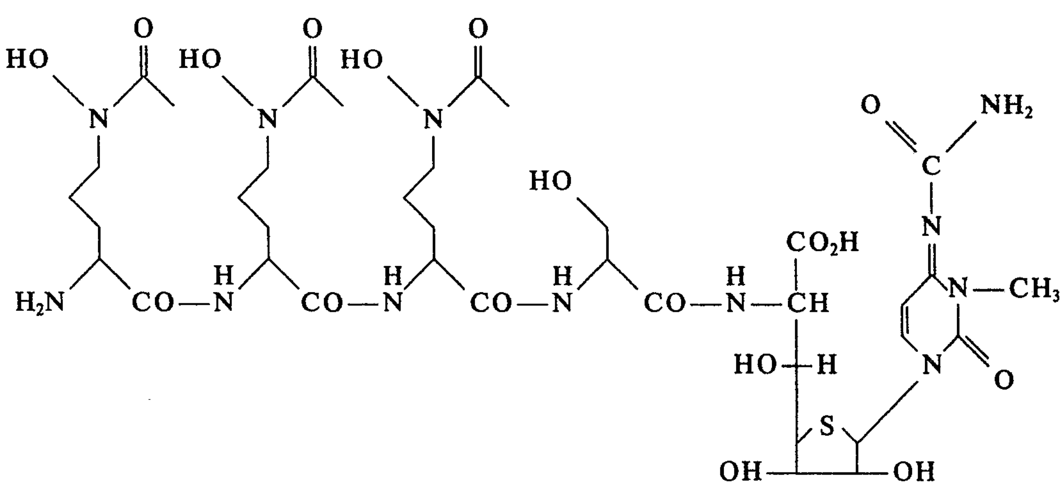

et son complexe de fer III.

2. Composé ayant un point de décomposition de 185°C, une rotation optique $[\alpha]_D^{20} = -27,7°$

(c = 0,2723 dans de l'eau, mesurée immédiatement après la dissolution), qui est aisément soluble dans l'eau à pH 7, notablement soluble dans le méthanol, la diméthylformamide et le diméthylsulfoxyde et difficilement soluble dans le chloroforme, l'éther, l'ester acétique et l'éther de pétrole, présente dans le spectre UV dans de l'eau (acide) un maximum à 304 nm, sous forme de comprimé avec du KBr dans la région infrarouge des maxima d'absorption à 3388, 2944, 1695, 1648, 1545, 1457, 1417, 1390, 1299, 1240, 1070, 1050 et 975 et libère par hydrolyse acide de l'ornithine et de la sérine dans le rapport 3 : 1, et son complexe de fer III.

3. Antibiotique selon l'une des revendications 1 ou 2, caractérisé par un spectre NMR 1 H selon la figure 2, par un spectre NMR 13C selon la figure 3 et par un maximum à 282 nm dans le spectre UV dans de l'eau à pH 7.

4. Procédé de fabrication d'un composé renfermant du fer ou exempt de fer selon l'une des revendications 1 à 3, caractérisé en ce qu'on cultive dans des conditions submergées et aérobies le Streptomyces spec. WS 116 (DSM 1692) dans un milieu nutritif contenant du carbone assimilable, de l'azote ainsi que des sels minéraux, en particulier des sels de fer, à une température de 15 à 35°C et en ce qu'on isole le composé formé à partir du liquide de fermentation.

5. Procédé selon la revendication 4, caractérisé en ce que pour l'isolation on fixe le composé conforme à l'invention sur une résine d'absorption non spécifique et on le désorbe avec des mélanges d'eau et d'un solvant organique.

6. Procédé selon les revendications 4 et 5, caractérisé en ce qu'on ajoute au bouillon de culture avant l'adsorption un sel de fer.

7. Procédé selon les revendications 4 à 6, caractérisé en ce que pour l'isolation on chromatographie l'antibiotique brut sur un échangeur de cations sous la forme $Fe^{3+}$, on élue les substances d'accompagnement inactives avec un tampon à concentration ionique élevée et l'on élue l'antibiotique avec le même tampon et avec addition d'un formateur de complexe pour le fer.

8. Procédé selon la revendication 7, caractérisé en ce qu'on chromatographie le produit obtenu selon la revendication 7 sur une colonne de CM-cellulose en la forme $H^+$ et on élue de manière fractionnée avec de l'eau.

9. Médicament ayant une teneur en composé selon l'une des revendications 1 à 3.

10. Composé selon l'une des revendications 1 à 3 dans l'utilisation pour combattre les infections bactériennes.

11. Procédé selon les revendications 4 à 8, caractérisé en ce qu'on fermente dans une solution nutritive que contient comme source principale de carbone de l'acide citrique, plusieurs sources d'azote complexes (extrait de levure, farine de fève de soya, eau de gonflement du maïs) et les aminoacides L-ornithine et L-sérine.

12. Procédé selon les revendications 4 à 8, caractérisé en ce qu'on fermente dans une solution nutritive entièrement synthétique qui, à côté de sels minéraux, contient comme source principale de carbone de l'acide citrique et comme unique source d'azote de la L-arginine.

13. Procédé selon les revendications 4 à 8, caractérisé en ce qu'à une solution nutritive, pour accroître le rendement, on alimente ultérieurement un mélange d'acide citrique-L-arginine et de sels à des taux pour maintenir une valeur de pH constante.

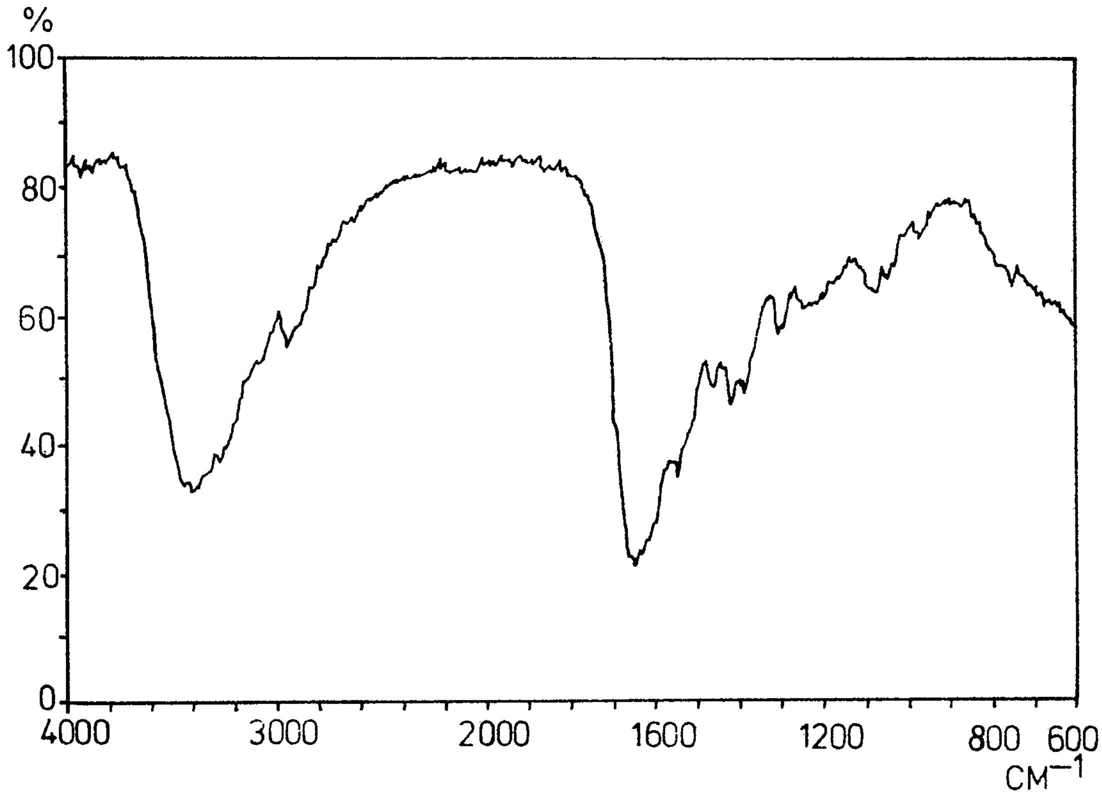


FIG. 1

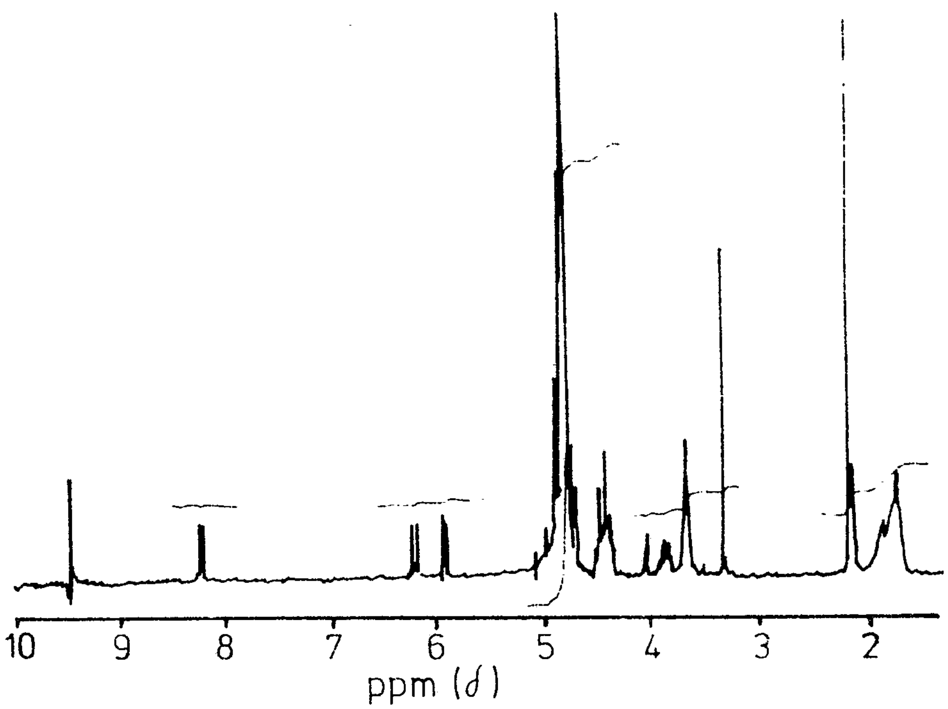


FIG. 2

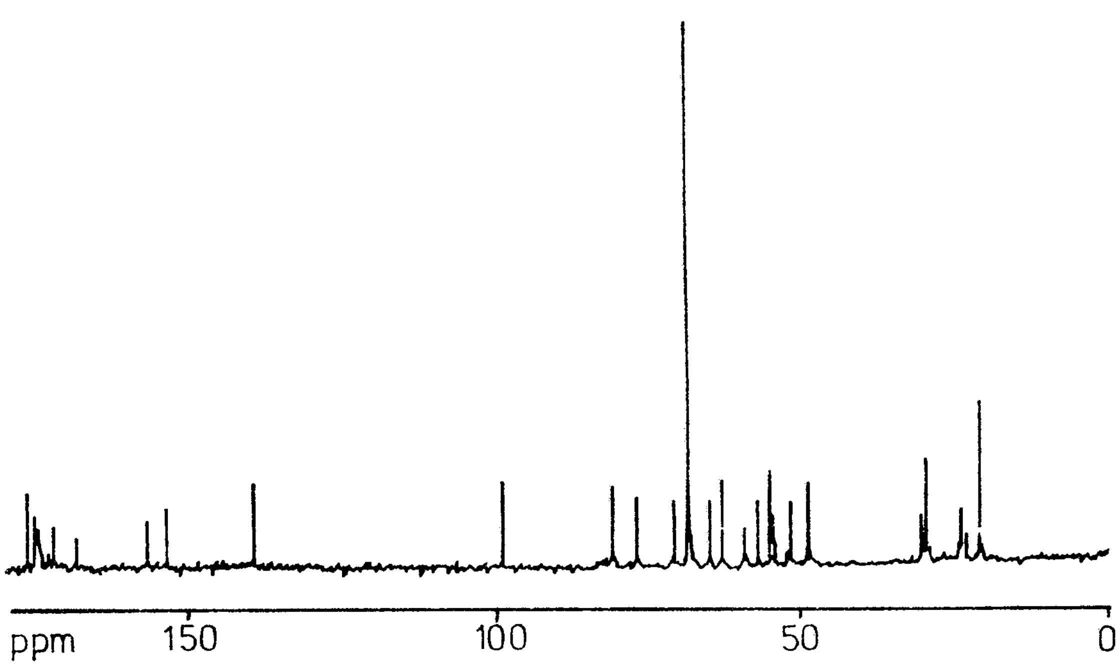


FIG. 3